# FASCICULE DE BREVET EUROPEEN

(11) **EP 2 688 651 B1**
(45) Date de publication et mention de la délivrance du brevet: **28.12.2016**
(21) Numéro de dépôt: 12717362.3
(22) Date de dépôt: 23.03.2012
(51) Int. Cl.: A61Q 19/00, A61K 8/73, A61K 8/92, A61K 8/37, A61K 8/34, A61K 9/70, A61P 17/02, A61L 26/00

(54) **COMPOSITION CONTENANT UNE CELLULOSE, UNE HUILE VEGETALE ET UN SOLVANT VOLATIL, SON UTILISATION COMME PANSEMENT**
ZUBEREITUNG ENTHALTEND EINE ZELLULOSE, EINEN PFLANZLICHEN ÖL UND EIN FLÜCHTIGES LÖSUNGSMITTEL, DEREN VERWENDUNG ALS PFLASTER
COMPOSITION COTAINING A CELLULOSE, A VEGETAL OIL AND A VOLATILE SOLVENT, ITS USE AS WOUND DRESSING

(30) Priorité: 25.03.2011 FR 1152513
(43) Date de publication de la demande: 29.01.2014
(73) Titulaire: URGO RECHERCHE INNOVATION ET DEVELOPPEMENT, 21300 Chenôve (FR)
(72) Inventeur: DERAIN, Nathalie, F-21370 Prenois (FR)
(74) Mandataire: Chantraine, Sylvie Hélène
(86) Numéro de dépôt international: PCT/FR2012/050604
(87) Numéro de publication internationale: WO 2012/131238

(56) Documents cités:
- GB-A- 754 844
- GB-A- 795 583
- US-A- 5 433 950
- US-A- 5 525 358
- US-A1- 2007 048 355

## Description

La présente invention concerne une composition fluide destinée à être appliquée sur la peau, contenant un dérivé de cellulose, une huile végétale et un solvant dudit dérivé qui est volatil. Une fois appliquée sur la peau, le solvant volatil s'évapore et la composition forme un film solide qui peut être avantageusement utilisé comme pansement d'une plaie.

Les pansements « liquides » peuvent être formulés en phase aqueuse ou en phase solvant. Ils se présentent en général sous la forme d'un fluide que l'on applique sur la peau par l'intermédiaire d'un applicateur approprié, tel un spray, un pinceau, une spatule ou une palette. L'eau ou le solvant qu'ils contiennent s'évapore au contact de la peau de manière à former un film solide qui protège la peau dont les constituants pénètrent de façon très limitée dans la peau. Ces pansements présentent l'avantage de protéger les tissus en évitant des contaminations bactériennes tout en les laissant respirer.

Les pansements liquides formulés à l'eau sont souvent longs à sécher. Ils se détruisent facilement dès que le corps entre en contact avec de l'eau, si bien qu'on leur préfère les pansements en phase solvant, dotés d'une meilleure résistance à l'eau mais aussi aux frottements et aux savons.

Des pansements liquides à base de nitrocellulose contenant une huile et un solvant volatil ont déjà été décrits dans l'art antérieur.

Par exemple, les formulations décrites dans la demande WO 2001/0037782 contiennent du Collodion, des huiles et des actifs. La Demanderesse a cependant constaté que la quantité de composition déposée sur la peau n'est pas suffisante pour recouvrir convenablement la peau sur une surface relativement grande, et former un pansement suffisamment épais pour protéger efficacement une peau lésée. L'exemple de ce document contient 4,4% de nitrocellulose, 2,6% d'huile de ricin, et 10% d'huile siliconée. La Demanderesse a pu constater que le film obtenu après enduction de cette formule au pinceau est très fin et friable. La surface de protection est médiocre et le film non homogène.

Le document JP 2008-273918 décrit une composition analogue contenant 2-10% nitrocellulose, 0,3-3% d'huile de ricin et une huile siliconée. La Demanderesse a mis en évidence que le pourcentage de plastifiant est trop bas pour que le film formé soit souple. Le film obtenu est cassant, a tendance à s'effriter et à se craqueler ; il est inesthétique et ne protège pas correctement la peau ou la plaie.

Enfin, le document US 2007/004835 concerne un pansement liquide pour petites plaies ou petites coupures, dans lequel il est proposé de remplacer une partie des solvants irritants pour la peau (utilisés de façon classique dans les pansements liquides) par des alcanes volatils. Un des exemples proposés contient 3,85% en poids de nitrocellulose et 2% d'huile de ricin. Ce document mentionne que la quantité de nitrocellulose peut varier de 3 à 7% en poids sec. La Demanderesse a pu constater que la nitrocellulose utilisée en faible quantité conduit à un film trop fin pour être protecteur.

Un pansement liquide prêt à l'emploi contenant de la nitrocellulose, de l'huile de ricin, de l'huile de germe de blé, de l'éthanol et de l'acétate d'éthyle a été commercialisé sous la référence Filmogel^{®} Crevasses. Il est appliqué à l'aide d'un pinceau ou d'une spatule afin de combler et de recouvrir uniformément une crevasse. Le produit est appliqué sur une zone très restreinte de la peau. Lorsque l'on essaie de l'appliquer sur une zone plus large, le film déposé est inhomogène, car la composition ne s'étale pas facilement.

La Demanderesse a donc souhaité développer un pansement liquide filmogène qui puisse recouvrir de façon satisfaisante une zone de peau plus étendue, tout en obtenant un film continu, souple, confortable et d'épaisseur suffisante. Le produit de l'invention peut être avantageusement appliqué sur une zone de la peau soumise à des mouvements des tensions ou des élongations, notamment au niveau des articulations.

La Demanderesse a découvert que les proportions de nitrocellulose et d'huile végétale doivent être suffisamment élevées pour que l'étalement du pansement liquide sur la peau sur une zone large soit homogène, et que le confort du film sec sur la peau, une fois le solvant évaporé, soit satisfaisant.

La Demanderesse a ainsi élaboré une nouvelle base de pansement dotée de résistances à l'eau et aux frottements satisfaisantes. Les films sont en outre souples et procurent un confort suffisant à l'utilisateur notamment lorsque le pansement est appliqué sur une zone assez étendue de la peau. Les pansements de l'invention peuvent être appliqués de façon homogène sur une zone d'application relativement étendue, typiquement supérieure à 1 cm², voir même supérieure à 5 cm². La quantité de produit déposée est suffisante pour recouvrir correctement la plaie.

La présente invention a donc pour objet une composition fluide destinée à être appliquée sur la peau, contenant un dérivé de cellulose, une huile végétale, un solvant dudit dérivé qui est volatil, caractérisée en ce que le dérivé de cellulose représente de 6 à 13% en poids sec, notamment de 6 à 12% en poids sec du poids total de la composition, en ce que l'huile végétale représente de 5 à 15% en poids du poids total de la composition, et en ce que le ratio massique huile/cellulose en poids sec est compris entre 0,8 et 1,5.

Le dérivé de cellulose peut être choisi parmi la nitrocellulose, l'hydroxypropylméthylcellulose, l'hydroxylpropylcellulose, l'éthylcellulose, les esters de cellulose, et leurs mélanges.

Les esters de cellulose peuvent être choisis parmi les acétates, les propionates, les butyrates, les isobutyrates, les acétobutyrates, les acétopropionates de cellulose et leurs mélanges.

Selon un mode de mise en oeuvre, le polymère est une nitrocellulose. La nitrocellulose est de préférence choisie parmi les nitrocelluloses de viscosité élevée, notamment les nitrocelluloses de qualité comprise entre RS 1/2 et RS 20 secondes selon la norme américaine qui correspond à une qualité comprise entre 8E et 21E selon la norme européenne. On préfère par exemple la nitrocellulose de qualité 10E ou 11E selon la norme européenne correspondant à la qualité RS 15 secondes selon la norme américaine.

La nitrocellulose peut être choisie notamment parmi les nitrocelluloses RS 5 sec. et RS 15 sec. commercialisées par la société HERCULES, les produits DHL^{®} 120/170, DHL^{®} 25/45 ou DHX^{®} 40/70 commercialisés par Nobel Enterprises, les nitrocelluloses E 840^{®} et E 620^{®} produites par Wolff Cellulosics, et les produits commercialisés sous les références E80^{®}, E70^{®}, E60^{®} et E40^{®} par SNPE-Bergerac.

La nitrocellulose peut être fournie sous forme sèche ou en solution dans un solvant tel que l'isopropanol ou l'éthanol.

La nitrocellulose est, de préférence, présente en une teneur allant de 6 % à 13 % en poids sec, de préférence de 9 à 11% en poids sec, par exemple entre 9,5 et 10,5% en poids sec par rapport au poids total de la composition.

La masse moléculaire Mw de la nitrocellulose est avantageusement comprise entre 60 000 et 80 000.

On entend par huile végétale, une huile issue d'une source végétale, qui avoir subi des traitements annexes de raffinage et/ou de purification, ou encore une transformation chimique, telle que l'estérification, l'hydrogénation, la peroxydation ou l'éthérification avec un autre composé chimique ou un autre corps gras naturel, par exemple.

L'huile végétale est avantageusement choisie parmi l'huile de sésame, l'huile de ricin, l'huile d'amande, l'huile de canola, l'huile de noisette, l'huile de pistache, l'huile de lin, l'huile de bourrache, l'huile de chanvre, l'huile de jojoba, l'huile de tournesol, l'huile de germe de blé, l'huile de maïs et/ou de germe de maïs, l'huile d'arachide, l'huile d'avocat, l'huile de carthame, l'huile de colza, l'huile d'olive, l'huile d'argan, l'huile de tournesol, l'huile de pépin de raisin, l'huile de soja, l'huile de noix, l'huile de pépin de de courge, l'huile de palme, l'huile de coprah, et leurs mélanges.

L'huile peut également être un dérivé d'une des huiles végétales citées précédemment. Il peut s'agir d'huile hydrogénée ou non, peroxydée ou non.

Elle est avantageusement soluble dans le solvant de la composition.

La quantité d'huile, en particulier d'huile de ricin, représente par exemple de 5 à 15 % en poids par rapport au poids total de la composition, avantageusement de 5 à 12 % en poids, de préférence de 8 à 12% en poids, de préférence encore de 9,5 à 10,5% en poids par rapport au poids total de la composition.

L'huile et la nitrocellulose peuvent être présentes dans la composition selon l'invention dans un rapport pondéral huile : nitrocellulose (en poids sec) compris entre 0,8 et 1,5, ou encore entre 0,9 et 1,25, plus particulièrement de l'ordre de 1.

Les compositions de l'invention contiennent également un solvant volatil, qui solubilise le dérivé de cellulose. Ce solvant permet de solubiliser une partie ou la totalité des ingrédients de la composition et participe à la formation d'un film sur la peau lors de son application.

Dans le cadre de la présente invention, on entend par solvant volatil un solvant capable de s'évaporer rapidement au contact de la peau. L'eau est exclue de cette définition. On préfère les solvants volatils ou les mélanges de solvant volatils non aqueux dont la température d'ébullition est supérieure à 50°C (à pression atmosphérique).

Comme solvant volatil utilisable dans le cadre de la présente invention, on peut citer :
- les cétones telles que la méthyléthylcétone, la méthylisobutylcétone, la diisobutylcétone, l'isophorone, la cyclohexanone, l'acétone;
- les alcools tels que l'éthanol, l'isopropanol, le n-propanol, le n-butanol, le diacétone alcool, le 2-butoxyéthanol, le cyclohexanol;
- les glycols tels que l'éthylène glycol, le propylène glycol, le pentylène glycol, le glycérol;
- les éthers de propylène glycol tels que le monométhyléther de propylène glycol, l'acétate de monométhyl éther de propylène glycol, le mono n-butyl éther de dipropylène glycol;
- les esters tels que l'acétate d'éthyle, l'acétate de méthyle, l'acétate de propyle, l'acétate de n-butyle, l'acétate d'isopentyle;
- les éthers tels que le diéthyléther, le diméthyléther ou le dichlorodiéthyléther; et
- leurs mélanges.

Le solvant volatil sera choisi avantageusement parmi l'éthanol, l'acétate d'éthyle et leurs mélanges. Selon un mode de mise en oeuvre, le solvant volatil est un mélange d'acétate d'éthyle et d'éthanol, préférentiellement dans des proportions massiques comprises entre 1/1 et 3/1, de préférence de l'ordre de 2/1.

On préfère des solvants ou des mélanges de solvant dont la vitesse d'évaporation est inférieure à celle des éthers. On pourra choisir des solvants ou des mélanges de solvant dont le profil d'évaporation est proche de celui du mélange d'acétate d'éthyle et d'éthanol précité.

La Demanderesse a en effet constaté que la composition s'étale mieux lorsque le solvant volatil a une vitesse d'évaporation inférieure à celle d'un mélange alcool/éther (25/75 volume/volume) tel que décrit dans l'art antérieur.

La quantité de solvant volatil représente par exemple de 60 à 90 % en poids par rapport au poids total de la composition, avantageusement de 70 à 90 % en poids par rapport au poids total de la composition.

La composition est essentiellement dépourvue d'eau. Elle en contient de préférence moins de 2% en poids.

Dans un mode de réalisation, la composition contient de 9 à 11% en poids sec de nitrocellulose, de 9 à 11% en poids d'huile de ricin, et un mélange d'un ester volatil et d'un alcool volatil.

Les compositions selon l'invention peuvent également contenir des additifs habituellement utilisés dans la préparation des pansements, comme les parfums, les conservateurs, les agents dépigmentants, les agents anti-bactériens, les agents antifongiques, les agents cicatrisants, les anti-douleurs, les anti-inflammatoires, les agents hydratants, les agents kératolytiques, les vitamines, la glycérine, l'acide citrique.

De manière générale, les actifs sont choisis parmi :
- les anti-bactériens tels que le Polymyxine B, les pénicillines (Amoxycilline), l'acide clavulanique, les tétracyclines, la Minocycline, la chlorotétracycline, les aminoglycosides, l'Amikacine, la Gentamicine, la Néomycine, l'argent et ses sels (Sulfadiazine argentique), les probiotiques ;
- les antiseptiques tels que le mercurothiolate de sodium, l'éosine, la chlorhexidine, le borate de phénylmercure, l'eau oxygénée, la liqueur de Dakin, le triclosan, le biguanide, l'hexamidine, le thymol, le Lugol, la Povidone iodée, le Merbromine, le Chlorure de Benzalkonium et de Benzethonium, l'éthanol, l'isopropanol ;
- les anti-viraux tels que l'Acyclovir, le Famciclovir, le Ritonavir ;
- les anti fongiques tels que les polyènes, le Nystatin, l'Amphotéricine B, la Natamycine, les imidazolés (Miconazole, Ketoconazole, Clotrimazole, Éconazole, Bifonazole, Butoconazole, Fenticonazole, Isoconazole, Oxiconazole, Sertaconazole, Sulconazole, Thiabendazole, Tioconazole), les triazolés (Fluconazole, Itraconazole, Ravuconazole, Posaconazole, Voriconazole), les allylamines, la Terbinafine, l'Amorolfine, la Naftifine, la Buténafine ;
- la Flucytosine (antimétabolite), la Griséofulvine, la Caspofungine, la Micafungine ;
- les anti-douleurs tels que le Paracétamol, la Codéine, le Dextropropoxyphène, le Tramadol, la Morphine et ses dérivés, les Corticoïdes et dérivés ;
- les anti-inflammatoires tels que les Glucocorticoïdes, les anti inflammatoires non stéroïdiens, l'Aspirine, l'Ibuprofène, le Kétoprofène, le Flurbiprofène, le Diclofénac, l'Acéclofénac, le Kétorolac, le Méloxicam, le Piroxicam, le Ténoxicam, le Naproxène, l'Indométacine, le Naproxcinod, le Nimésulide, le Célécoxib, l'Etoricoxib, le Parécoxib, le Rofécoxib, le Valdécoxib, la Phénylbutazone, l'acide niflumique, l'acide méfénamique, l'acide bêta-18-glycyrrhétinique ;
- les actifs favorisant la cicatrisation tels que le Rétinol, la Vitamine A, la Vitamine E, la N-acétyl-hydroxyproline, les extraits de *Centella Asiatica,* la papaïne, les silicones, les huiles essentielles de thym, de niaouli, de romarin et de sauge, l'acide hyaluronique, les oligosaccharides polysulfatés synthétiques ayant 1 à 4 unités oses tels que le sel de potassium du sucrose octasulfaté, le sel d'argent du sucrose octasulfaté ou le sucralfate, l'Allantoïne ;
- les agents dépigmentants tels que l'acide kojique (Kojic Acid SL^{®} - Quimasso (Sino Lion)), l'Arbutine (Olevatin^{®} - Quimasso (Sino Lion)), le mélange de palmitoylpropyl de sodium et d'extrait de nénuphar blanc (Sepicalm^{®} - Seppic), l'undécylénoyl phénylalanine (Sepiwhite^{®} - Seppic), l'extrait de réglisse obtenue par fermentation *d'Aspergillus* et éthoxydiglycol (Gatuline Whitening^{®} - Gattefossé), l'acide octadécènedioïque (ODA White^{®} - Sederma), l'alpha-arbutin (Alpha-arbutin^{®}, SACI-CFPA (Pentapharm)), l'extrait aqueux de feuilles *Arctophylos Uva Ursi* (Melfade-J^{®} - SACI-CFPA (Pentapharm)), le mélange de plante complexe Gigawhite^{®} (SACI-CFPA (Alpaflor)), la diacétyl boldine (Lumiskin^{®} - Sederma), l'extrait de mandarine du Japon (Melaslow^{®} - Sederma), le mélange d'extrait de citron enrichi en acide citrique et d'extrait de concombre (Uninontan^{®}U-34 - Unipex), le mélange d'extrait de *Rumex occidentalis* et de vitamine C (Tyrostat^{®} 11 - Unipex), des oligopeptides (Mélanostatine 5^{®} - Unipex), le dipalmitate kojique (KAD-15^{®} - Quimasso (Sino Lion)), le complexe d'origine naturelle Vegewhite^{®} de LCW, des extraits de germe de blé (Clariskin^{®} II - Silab), l'éthyldiamine triacetate (EDTA);
- les agents kératolytiques tels que l'acide salicylique, le salicylate de zinc, l'acide ascorbique, les acides alpha hydroxylés (acide glycolique, lactique, malique, citrique, tartrique), les extraits d'Erable argenté, de Griottier, de Tamarinier, l'urée, le rétinoïde topique Kératoline^{®} (Sederma), les protéases obtenues par fermentation de *Bacillus Subtilis,* le produit Linked-Papain^{®} (SACI-CFPA), la papaïne (enzyme protéolytique issue du fruit de papaye) ;
- les actifs restructurants (par exemple resctructurants des phanères) tels que les dérivés de silice, la vitamine E, la camomille, le calcium, l'extrait de prêle, le Lipester de soie ;
- les filtres solaires, tels que les filtres chimiques (Oxybenzone, Sulisobenzone, Dioxybenzone, Tinosorb S^{®}, Avobenzone, p-méthoxycinnamate de 2-éthoxyéthyle, Uvinul^{®} A+, Mexoryl^{®} XL, Méthoxycinnamate ou octinoxate d'octyle, Salicylate ou octisalate d'octyle, octyl triazone ou Uvinul^{®} T 150, salicylate de méthyle, meradimate, enzacamène, MBBT ou Tinosorb^{®} M, cyanophénylcinnamate d'octyle ou Parsol^{®} 340, Acide para-aminobenzoïque, Ensulizole, Parsol^{®} SLX ou Polysiloxane-15 ou Benzylidène malonate polysiloxane, salicylate de triéthanolamine ou salicylate de trolamine, Mexoryl^{®} SX ou acide téréphtalylidène dicamphosulfonique;) et les filtres minéraux (oxydes de Zinc, dioxyde de titane, kaolin, ichtyol);
- les anesthésiques tels que la benzocaine, la lidocaïne, la dibucaïne, le chlorhydrate de pramoxine, la bupivacaïne, la mepivacaïne, la prilocaïne, l'étidocaïne.

La composition selon l'invention peut également contenir des arômes, des agents d'amertume (tels que le benzoate de dénatonium).

Comme indiqué précédemment, la composition selon la présente invention se présente sous forme d'un liquide fluide destiné à être appliqué à l'aide d'un applicateur approprié, tel qu'un pinceau ou une palette.

Les compositions selon la présente invention sont destinées à être appliquées sur des plaies ou des cicatrices, qu'elles soient liées à un accident, une maladie ou les suites d'une intervention chirurgicale, ou des brûlures. Ces compositions peuvent également être appliquées à toutes affections de la peau. On peut citer, à titre d'exemple, l'acné, la varicelle, le zona, la couperose, les brûlures au premier degré, l'eczéma, l'hyperpigmentation, la lucite, le vitiligo, la xérose, la prophyrie, les vergetures, le psoriasis, les piqûres d'insectes.

L'invention a donc pour objet la composition telle que définie ci-dessus pour son utilisation dans la protection des brûlures, en particulier les brûlures légères, des plaies cutanées superficielles, ou des cicatrices. Les compositions selon l'invention sont également utiles pour les traitements des ampoules, des gerçures, ou des crevasses. La composition selon la présente invention peut aussi être appliquée sur des blessures non totalement cicatrisées à condition qu'elles ne soient pas suintantes et qu'elles aient été préalablement décontaminées.

L'invention concerne également l'utilisation d'une composition telle que définie ci-dessus pour la préparation d'un pansement destiné à protéger les brûlures, les plaies cutanées superficielles ou les cicatrices.

Les exemples suivants illustrent l'invention, sans en limiter la portée.

### Exemple 1 :

On prépare la composition suivante.

| **Nom commercial** | **Fabricant/ Fournisseur** | **Dénomination INCI** | **n° CAS** | **% massique** |
|---|---|---|---|---|
| 1. DHL^{®} 120/170 IPA | Nobel | Nitrocellulose | 9004-70-0 | 11,5 |
| 2. Huile de ricin | Prod'hyg Laboratoire | RicinusCommunis (Castor) Seed Oil | 8001-79-4 | 10,0 |
| 3. Ethanol absolu | Charbonneau Brabant | Alcohol | 64-17-5 | 25,7 |
| 4. Acétate d'éthyle | Darfeuille | Ethylacetate | 141-78-6 | 51,3 |
| 5. Enoxolone^{®} PH | Cognis | Acide bêta 18 glycyrrhétinique | 471-53-4 | 1,5 |

### Mode opératoire:

On mélange les ingrédients 2 à 5 sous agitation à 700 tours/min pendant 10 minutes avec un agitateur à hélice. La nitrocellulose est ensuite dispersée dans le mélange sous agitation, et le mélange est agité successivement pendant 20 minutes à 1100 tours/min et pendant 30 minutes à 2000 tours/min.

### Test de tenue :

Les essais sont réalisés sur 13 personnes pendant 24h après une seule application, dans les conditions suivantes:
- le filmogène est déposé sur le front, le dessus de la main et la ceinture
- douche est obligatoire à T12h et facultative à T24h
- les évaluations sont conduites à T6h, T12h (avant douche et après douche) et T24h

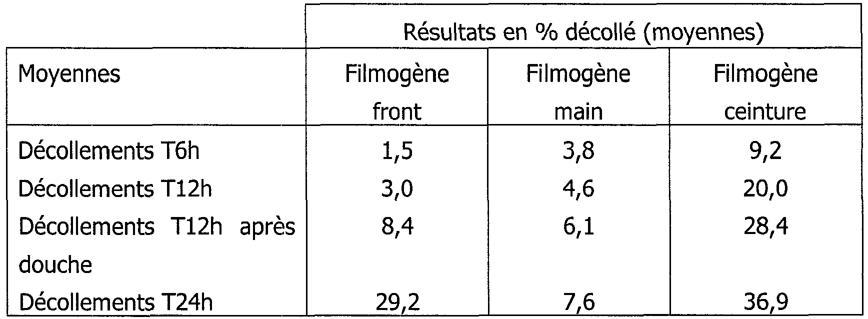

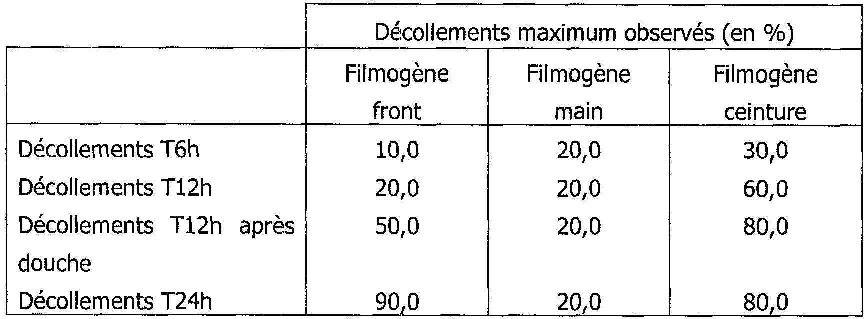

La tenue du filmogène sur la main est supérieure à la tenue du filmogène sur le front et la ceinture, ceci malgré les lavages de mains. La composition selon l'invention a donc une bonne résistance à l'eau.

Dès T6h de port, le filmogène appliqué à la ceinture est significativement plus décollé que sur les zones des mains et du front. Le décollement du filmogène sur le front est sans doute favorisé par les frottements des draps pendant la nuit puisqu'il passe de 8% en moyenne à T12h après douche à environ 30% en moyenne à T24h

Le filmogène sur main ne présente pas de décollements importants à T24h.

### Exemple 2 comparatif : produit Filmogel^{®} Crevasses

La composition de l'exemple 1 est étalée de façon homogène et calibrée sur un support inerte, puis on la laisse sécher afin que le solvant volatil s'évapore. On obtient un film de 30 microns d'épaisseur que l'on sépare du support.

Des éprouvettes de 15* 50mm (correspondant à une surface de 7,5 cm²) sont découpées pour l'évaluation. Elles sont soumises à un étirement, par un dynamomètre, qui va mesurer la longueur d'élongation maximale avant rupture et la force nécessaire.

Les mêmes éprouvettes sont préparées avec le produit Filmogel® Crevasses.

Les résultats obtenus sont :

| | |
|---|---|
| Filmogel® Crevasses = 43% d'élongation | Force nécessaire = 14 N/cm |
| Notre formule = 115% d'élongation | Force nécessaire = 7 N/cm |

La formule selon l'invention est: donc plus souple.

Le Filmogel^{®} Crevasses s'étale moins facilement car il est plus visqueux et contient peu de plastifiant, il est de ce fait moins glissant. Le film formé avec le Filmogel^{®} Crevasses est plus épais et moins souple. Aussi, le film est plus cassant et moins confortable.

### Exemple 3 comparatif :

| **Nom commercial** | **Fabricant/ Fournisseur** | **Dénomination INCI** | **n° CAS** | **% massique** |
|---|---|---|---|---|
| 1. DHL^{®} 120/170 IPA | Nobel | Nitrocellulose | 9004-70-0 | 17,6 |
| 2. Huile de ricin | Prod'hyg Laboratoire | RicinusCommunis (Castor) Seed Oil | 8001-79-4 | 15,3 |
| 3. Ethanol absolu | Charbonneau Brabant | Alcohol | 64-17-5 | 21,6 |
| 4. Acétate d'éthyle | Darfeuille | Ethylacetate | 141-78-6 | 43,3 |
| 5. Parsol^{®} 1789 | DSM | ButylMethoxydibenzoylmethane | 70356-09-1 | 1,0 |
| 6. Parsol^{®} MCX | DSM | EthylhexylMethoxycinnamate | 5466-77-3 | 1,2 |

### Mode opératoire:

On a mélangé les ingrédients 2 à 5 sous agitation à 700 tours/min pendant 10 minutes avec un agitateur à hélice. La nitrocellulose a ensuite été dispersée dans le mélange sous agitation, et le mélange a été agité successivement pendant 20 minutes à 1100 tours/min et pendant 30 minutes à 2000 tours/min.

La teneur en nitrocellulose de l'exemple 3 comparatif est de 14,1% en poids sec. La composition selon l'exemple 3 était très visqueuse et filante ce qui la rendait difficile à étaler au pinceau. De plus, le film formé par cette composition était long à sécher et n'était pas régulier. Ainsi, si l'on ajoute un (des) actif(s) ou un(des) filtre(s) solaire(s) à ce type de composition, la quantité d'actif(s) disponible ou la protection scolaire n'est pas homogène.

## Revendications

1. Composition fluide destinée à être appliquée sur la peau, contenant un dérivé de cellulose, une huile végétale, un solvant dudit dérivé qui est volatil, **caractérisée en ce que** le dérivé de cellulose représente de 6 à 13% en poids sec du poids total de la composition, **en ce que** l'huile végétale représente de 5 à 15% en poids du poids total de la composition, et **en ce que** le ratio massique huile/cellulose en poids sec est compris entre 0,8 et 1,5.

2. Composition selon la revendication 1, **caractérisée en ce que** le dérivé de cellulose est une nitrocellulose.

3. Composition selon l'une des revendications précédentes, **caractérisée en ce que** l'huile végétale est l'huile de ricin.

4. Composition selon l'une des revendications 1 à 3, **caractérisée en ce que** l'huile représente entre 7 et 12 % en poids du poids total de la composition.

5. Composition selon l'une des revendications 1 à 4, **caractérisée en ce que** le solvant dudit dérivé de cellulose représente entre 70 et 90 % en poids du poids total de la composition.

6. Composition selon l'une des revendications précédentes, **caractérisée en ce que** le solvant dudit dérivé de cellulose est choisi parmi l'éthanol, l'acétate d'éthyle et leurs mélanges.

7. Composition selon l'une des revendications précédentes, **caractérisée en ce qu'**elle comprend de la nitrocellulose, de l'huile de ricin, de l'acétate d'éthyle et de l'éthanol.

8. Composition selon l'une des revendications précédentes, **caractérisée en ce qu'**elle comprend en outre un ingrédient choisi parmi les agents dépigmentants, les agents anti-bactériens, les agents antifongiques, les agents cicatrisants, les anti-douleurs, les anti-inflammatoires, les agents hydratants, les agents kératolytiques, les vitamines, les agents restructurants des phanères, les filtres solaires et leurs mélanges.

9. Composition selon l'une des revendications 1 à 8, **caractérisée en ce qu'**elle est conditionnée dans un flacon doté d'un moyen d'application tel qu'un pinceau ou une spatule.

10. Composition selon l'une des revendications 1 à 8 pour son utilisation dans la protection des brûlures, des plaies, des cicatrices ou des affections de la peau.

## Patentansprüche

1. Fluide Zusammensetzung, welche dazu bestimmt ist, auf die Haut aufgebracht zu werden, wobei diese ein Cellulose-Derivat, ein pflanzliches Öl, ein Lösungsmittel des Derivats, das flüchtig ist, enthält, **dadurch gekennzeichnet, dass** das Cellulose-Derivat 6 bis 13 %, bezogen auf das Trockengewicht, des Gesamtgewichts der Zusammensetzung repräsentiert, dass das pflanzliche Öl 5 bis 15 %, bezogen auf das Gewicht, des Gesamtgewichts der Zusammensetzung repräsentiert, und dass das Massenverhältnis Öl/Cellulose, bezogen auf das Trockengewicht, zwischen 0,8 und 1,5 beträgt.

2. Zusammensetzung nach Anspruch 1, **dadurch gekennzeichnet, dass** das Cellulose-Derivat Nitrocellulose ist.

3. Zusammensetzung nach einem der vorhergehenden Ansprüche, **dadurch gekennzeichnet, dass** das pflanzliche Öl Rizinusöl ist.

4. Zusammensetzung nach einem der Ansprüche 1 bis 3, **dadurch gekennzeichnet, dass** das Öl zwischen 7 und 12 %, bezogen auf das Gewicht, des Gesamtgewichts der Zusammensetzung repräsentiert.

5. Zusammensetzung nach einem der Ansprüche 1 bis 4, **dadurch gekennzeichnet, dass** das Lösungsmittel des Cellulose-Derivats zwischen 70 und 90 %, bezogen auf das Gewicht, des Gesamtgewichts der Zusammensetzung repräsentiert.

6. Zusammensetzung nach einem der vorhergehenden Ansprüche, **dadurch gekennzeichnet, dass** das Lösungsmittel des Cellulose-Derivats aus Ethanol, Ethylacetat und ihren Mischungen ausgewählt ist.

7. Zusammensetzung nach einem der vorhergehenden Ansprüche, **dadurch gekennzeichnet, dass** diese Nitrocellulose, Rizinusöl, Ethylacetat und Ethanol umfasst.

8. Zusammensetzung nach einem der vorhergehenden Ansprüche, **dadurch gekennzeichnet, dass** diese außerdem einen Bestandteil umfasst, der aus Depigmentierungsmitteln, antibakteriellen Mitteln, antifungalen Mitteln, wundheilenden Mitteln, Schmerzmitteln, entzündungshemmenden Mitteln, Hydratisierungsmitteln, keratolytischen Mitteln, Vitaminen, Mitteln zur Restrukturierung der Hautanhangsgebilde, Sonnenfiltern und ihren Mischungen ausgewählt ist.

9. Zusammensetzung nach einem der Ansprüche 1 bis 8, **dadurch gekennzeichnet, dass** diese in einem Fläschchen abgefüllt ist, das mit einem Mittel zum Aufbringen, wie einem Pinsel oder einem Spachtel, versehen ist.

10. Zusammensetzung nach einem der Ansprüche 1 bis 8 für deren Verwendung beim Schützen von Verbrennungen, Wunden, Narben oder Hautkrankheiten.

## Claims

1. A fluid composition intended to be applied on skin, comprising a cellulose derivative, a vegetable oil and a solvent for said derivative which is volatile, **characterized in that** the cellulose derivative represents from 6% to 13% by dry weight of the total weight of the composition, **in that** the vegetable oil represents from 5% to 15% by weight of the total weight of the composition and **in that** the oil/cellulose ratio by weight, by dry weight, is between 0.8 and 1.5.

2. The composition as claimed in claim 1, **characterized in that** the cellulose derivative is a nitrocellulose.

3. The composition as claimed in either of the preceding claims, **characterized in that** the vegetable oil is castor oil.

4. The composition as claimed in one of claims 1 to 3, **characterized in that** the oil represents between 7% and 12% by weight of the total weight of the composition.

5. The composition as claimed in one of claims 1 to 4, **characterized in that** the solvent for said cellulose derivative represents between 70% and 90% by weight of the total weight of the composition.

6. The composition as claimed in one of the preceding claims, **characterized in that** the solvent for said cellulose derivative is chosen from ethanol, ethyl acetate and their mixtures.

7. The composition as claimed in one of the preceding claims, **characterized in that** it comprises nitrocellulose, castor oil, ethyl acetate and ethanol.

8. The composition as claimed in one of the preceding claims, **characterized in that** it additionally comprises an ingredient chosen from depigmenting agents, antibacterial agents, antifungal agents, healing agents, pain killers, anti-inflammatories, hydrating agents, keratolytic agents, vitamins, restructuring agents for superficial body growths, sunscreens and their mixtures.

9. The composition as claimed in one of claims 1 to 8, **characterized in that** it is packaged in a bottle having an application means, such as a brush or a spatula.

10. The composition as claimed in one of claims 1 to 8 for the use thereof in the protection of burns, wounds, scars or skin ailments.
